# EUROPEAN PATENT SPECIFICATION

(11) **EP 3 972 698 B1**
(45) Date of publication and mention of the grant of the patent: **12.02.2025**
(21) Application number: 20727722.9
(22) Date of filing: 18.05.2020
(51) Int. Cl.: A61Q 11/00, A61K 8/41, A61K 8/60

(54) **ORAL COMPOSITIONS AND METHODS OF USE**
ORALE ZUSAMMENSETZUNGEN UND VERFAHREN ZUR VERWENDUNG
COMPOSITIONS ORALES ET LEURS MÉTHODES D'UTILISATION

(30) Priority: 22.05.2019 US 201962851526 P
(43) Date of publication of application: 30.03.2022
(73) Proprietor: Solventum Intellectual Properties Company, Maplewood, MN 55144 (US)
(72) Inventor: RUSIN, Richard P., Saint Paul, Minnesota 55133-3427 (US); YANG, Jie, Saint Paul, Minnesota 55133-3427 (US); KOHLER RIEDI, Petra L., Saint Paul, Minnesota 55133-3427 (US); CONNELL, Jodi L., Saint Paul, Minnesota 55133-3427 (US); HAEBERLEIN, Ingo R., 82229 Seefeld (DE); COHEN, Hannah C., Saint Paul, Minnesota 55133-3427 (US); WLASCHIN, Katie F., Saint Paul, Minnesota 55133-3427 (US); KLAIBER, Paul R., Saint Paul, Minnesota 55133-3427 (US); WANG, Yizhong, Saint Paul, Minnesota 55133-3427 (US); OXMAN, Joel D., Saint Paul, Minnesota 55133-3427 (US); TON, Tiffany T., Saint Paul, Minnesota 55133-3427 (US); BOEHMER, Roxanne A., Saint Paul, Minnesota 55133-3427 (US)
(74) Representative: Vossius & Partner Patentanwälte Rechtsanwälte mbB
(86) International application number: PCT/IB2020/054685
(87) International publication number: WO 2020/234744

(56) References cited:
- WO-A1-2004/058257
- WO-A1-2009/014905
- WO-A1-2017/205230
- WO-A1-2019/106521
- WO-A1-2019/123171
- WO-A1-2020/079531
- WO-A2-2008/033911
- US-A1- 2010 150 847
- DATABASE GNPD [online] MINTEL; 28 October 2014 (2014-10-28), ANONYMOUS: "Flirt Mascara", XP055714987, retrieved from www.gnpd.com Database accession no. 2750667

## Description

### TECHNICAL FIELD

The present disclosure relates to oral compositions, and more specifically oral compositions and their use for inhibiting biofilm formation in the oral cavity of a subject.

### BACKGROUND

Dental plaque, which may include bacteria such as *Streptococcus mutans,* for example comprises a biofilm that forms on surfaces in the oral cavity. Dental plaque is at least partly responsible for dental caries, gingivitis, and periodontal diseases. Bacteria in dental plaque metabolize carbohydrates (for example, simple sugars) in the mouth and produce acids that can etch tooth enamel. The dentin thus exposed can then be colonized by bacteria. Dental plaque can serve as a substrate for the deposition of tartar or calculus. Build-up of calculus can lead to gingivitis and, ultimately, to periodontal disease. A currently available method to remove dental plaque from teeth is mechanical removal with, for example, dental floss or a toothbrush. A toothbrush can aid in removing dental plaque from exposed surfaces of a tooth, and dental floss can aid in removing dental plaque from, for example, interproximal and subgingival surfaces. Proper and regular use of dental floss and a toothbrush can mechanically remove or reduce dental plaque, and can reduce the incidence of dental caries, gingivitis, and periodontal disease. Regular visits to a dentist or hygienist to receive professional prophylaxis can also help to reduce such oral maladies. Certain antimicrobial formulations are available (in the form of mouthwashes, rinses, and toothpastes, for example) to aid in the control and treatment of dental plaque, dental caries, gingivitis, and periodontal disease. Therapeutic dental compositions and related methods to inhibit biofilm formation have been described in U.S. Pat. No. 8,968,709 (Yang et al.).

The formation of biofilms can also be problematic in subjects who suffer from xerostomia or dry mouth that results from insufficient saliva volume.
WO 2009/014905 A1 discloses film forming dental compositions and methods to inhibit biofilm formation in the oral cavity of a subject. WO 2017/205230 A1 discloses dental pastes and associated methods and kits to inhibit biofilm formation in the oral cavity of a subject. WO 2019/123171 A1 is prior art under Art. 54(3) EPC and discloses oral compositions and methods to relieve xerostomia and improve oral health of a subject.

### SUMMARY

The present invention is defined in the appended claims.

Disclosed herein are compositions that include; from 1 wt-% to 3 wt-% total of glucosamine, a derivative of glucosamine, or a combination thereof based on the total weight of the composition; and from 0.3 wt-% to 3 wt-% total of one or more surfactants, based on the total weight of the composition, of Formula I:

HOCH₂-(CHOH)ₙ-CH₂NR¹R² (I)

wherein R¹ and R² are independently selected from the group consisting of a hydrogen atom, an alkyl group, C(O)R³, and SO₂R⁴; with R³ and R⁴ being independently selected from the group consisting of an alkyl group, an aryl group, and an aralkyl group; wherein n is an integer from 2 to 5 with the proviso that Aqueous Solution A is excluded.

In another aspect, the above composition for use in treating a surface in the oral cavity of a subject is provided in claim 13. The method can include: (a) providing the dental paste; and (b) applying the dental paste to a surface in the oral cavity of the subject.

In another aspect, the above composition for use in inhibiting biofilm formation on a surface in the oral cavity of a subject is provided in claim 12. The use can include: (a) providing the dental paste; and (b) applying the dental paste to a surface in the oral cavity of the subject.

The above summary is not intended to describe each embodiment of the present disclosure. The details of one or more embodiments of the present disclosure are also set forth in the description below. Other features, objects, and advantages of the present disclosure will be apparent from the description and from the claims.

### DETAILED DESCRIPTION

Disclosed herein are compositions that can be utilized as oral compositions, for example. Disclosed compositions include both a particular type of surfactant(s) and glucosamine, a derivative(s) thereof, or combinations thereof. Disclosed compositions may have effects on the formation of biofilms. In some embodiments, disclosed compositions may have effects on the formation of biofilms that are better and in some embodiments exhibit a synergistic effect when compared to compositions with the particular type of surfactant and compositions with glucosamine or derivative(s) thereof.

Disclosed compositions, one or more components in a composition, or both can be characterized as edible. Referring to a component or composition as edible can mean that the particular ingredient or composition is safe for daily, long-term ingestion at recommended use levels. In some embodiments, the GRAS (generally regarded as safe) list from the United States Food and Drug Administration (FDA) can be utilized to determine if a component is edible at the levels utilized in a composition.

Disclosed compositions include at least glucosamine or a derivative(s) thereof, and a surfactant of Formula I:

HOCH₂-(CHOH)ₙ-CH₂NR¹R² (I)

wherein R¹ and R² are independently selected from the group consisting of a hydrogen atom, an alkyl group, C(O)R³, and SO₂R⁴; with R³ and R⁴ being independently selected from the group consisting of an alkyl group, an aryl group, and an aralkyl group; and n is an integer from 2 to 5.

Disclosed compositions include glucosamine or a derivative thereof. Glucosamine can also be referred to as (3R,4R,5S)-3-Amino-6-(hydroxymethyl)oxane-2,4,5-triol, or 2-amino-2-deoxy-glucose. Derivatives of glucosamine include salts of glucosamine for example. Commonly utilized salt forms of glucosamine include, for example glucosamine sulfate, and glucosamine hydrochloride. Another common derivative of glucosamine is N-acetylglucosamine, which is an amide formed from glucosamine and acetic acid. Any combination of glucosamine and derivatives thereof can be utilized in disclosed compositions. In the present invention, the derivative of glucosamine is selected from salts of glucosamine and N-acetylglucosamine.

Disclosed compositions include not greater than 3 wt-% of glucosamine or a derivative(s) thereof based on the total weight of the composition, not greater than 2.5 wt- % of glucosamine or a derivative(s) thereof based on the total weight of the composition, or not greater than 2.2 wt-% of glucosamine or a derivative thereof based on the total weight of the composition. Disclosed compositions include not less than 1 wt-% of glucosamine or a derivative(s) thereof based on the total weight of the composition, not less than 1.2 wt-% of glucosamine or a derivative(s) thereof based on the total weight of the composition, or not less than 1.5 wt-% of glucosamine or a derivative(s) thereof based on the total weight of the composition.

Disclosed compositions include surfactants of Formula I:

HOCH₂-(CHOH)ₙ-CH₂NR¹R² (I)

wherein R¹ and R² are independently selected from the group consisting of a hydrogen atom, an alkyl group, C(O)R³, and SO₂R⁴; with R³ and R⁴ being independently selected from the group consisting of an alkyl group, an aryl group, and an aralkyl group; and n is an integer from 2 to 5. The groups R¹ and R² are independently selected from the group consisting of a hydrogen atom, an alkyl group, C(O)R³, and SO₂R⁴.

In Formula I, each of R¹ and R² may be a hydrogen atom, each of R¹ and R² may be an alkyl group, each of R¹ and R² may be C(O)R³, or each of R¹ and R² may be SO₂R⁴. In some embodiments, R¹ may be a hydrogen atom and R² may be an alkyl group, C(O)R³, or SO₂R⁴. In other embodiments, R¹ may be an alkyl group, and R² may be C(O)R³, or SO₂R⁴. In still other embodiments, R¹ may be C(O)R³, and R² may be SO₂R⁴. When either or both of R¹ and R² is an alkyl group, the alkyl group may comprise about one carbon atom, more than about one carbon atom, more than about two carbon atoms, more than about four carbons atoms, more than about six carbon atoms, more than about eight carbon atoms, more than about ten carbon atoms, more than about twelve carbon atoms, more than about fourteen carbon atoms, more than about sixteen carbon atoms, or more than about eighteen carbon atoms. In some embodiments, the alkyl group comprises less than about thirty carbon atoms, less than about twenty-six carbon atoms, or less than about twenty carbon atoms. For example, when R¹ is an alkyl group, R¹ can be a C₁-C₁₂ alkyl group, a C₁-C₁₀ alkyl group, C₁-C₆ alkyl group, or a C₂-C₇ alkyl group. In particular embodiments, R¹ is a hydrogen atom or a C₁-C₂ alkyl group. In some embodiments, the alkyl group comprises a straight chain alkyl group. In other embodiments, the alkyl group comprises a branched alkyl group. In still other embodiments, the alkyl group comprises a cyclic alkyl group. When each of R¹ and R² comprises an alkyl group, R¹ and R² may comprise the same alkyl group, or R¹ and R² may comprise different alkyl groups. Non-limiting examples of alkyl groups include methyl, ethyl, 1-propyl, iso-propyl, butyl, iso-butyl, sec-butyl, pentyl, iso-pentyl, neo-pentyl, hexyl, 2-ethylhexyl, octyl, decyl, undecyl, dodecyl, tetradecyl, pentadecyl, octadecyl, cyclohexyl, 4-methylcyclohexyl, cyclohexylmethyl, cyclopentyl, and cyclooctyl.

The groups R³ and R⁴ are independently selected from the group consisting of an alkyl group, an aryl group, and an aralkyl group. When either or both of R³ or R⁴ is an alkyl group, the alkyl group may comprise about one carbon atom, more than about one carbon atom, more than about two carbon atoms, more than about four carbons atoms, more than about six carbon atoms, more than about eight carbon atoms, more than about ten carbon atoms, more than about twelve carbon atoms, more than about fourteen carbon atoms, more than about sixteen carbon atoms, or more than about eighteen carbon atoms. In some embodiments, the alkyl group comprises less than about thirty carbon atoms, less than about twenty-six carbon atoms, or less than about twenty carbon atoms. In some embodiments, the group R³ is a C₇-C₉ alkyl group, for example, an alkyl group including about seven carbon atoms (i.e., C₇), about eight carbon atoms (i.e., Cs), or about nine carbon atoms (i.e., C₉). In some embodiments, the alkyl group comprises a straight chain alkyl group. In other embodiments, the alkyl group comprises a branched alkyl group. In still other embodiments, the alkyl group comprises a cyclic alkyl group. In compounds of Formula I or pharmaceutically acceptable salts thereof, when both R³ and R⁴ groups are present, and when each of R³ and R⁴ comprises an alkyl group, R³ and R⁴may comprise the same alkyl group, or R³ and R⁴ may comprise different alkyl groups. Non-limiting examples of alkyl groups include methyl, ethyl, 1-propyl, iso-propyl, butyl, iso-butyl, sec-butyl, pentyl, iso-pentyl, neo-pentyl, hexyl, 2-ethylhexyl, octyl, decyl, undecyl, dodecyl, tetradecyl, pentadecyl, octadecyl, cyclohexyl, 4-methylcyclohexyl, cyclohexylmethyl, cyclopentyl, and cyclooctyl.

When either or both of R³ or R⁴ are an aryl group, the aryl group may comprise one arene ring or more than one arene ring. Arene rings may comprise up to six carbon atoms, up to eight carbon atoms, up to ten carbon atoms, up to twelve carbon atoms, up to fourteen carbon atoms, up to sixteen carbon atoms, or up to eighteen carbon atoms. Arene rings may comprise a heteroatom, for example, nitrogen, oxygen, or sulfur. If more than one arene ring is present, the arene rings may be fused together, or they may be joined by a chemical bond. In compounds of Formula I or pharmaceutically acceptable salts thereof, when both R³ and R⁴ groups are present, and when each of R³ and R⁴ comprises an aryl group, R³ and R⁴ may comprise the same aryl group, or R³ and R⁴ may comprise different aryl groups. Non-limiting examples of aryl groups include substituted and unsubstituted phenyl, 1-naphthyl, 2-naphthyl, 9-anthracenyl.

When either or both of R³ or R⁴ are an aralkyl group, the aralkyl group may comprise one arene ring or more than one arene ring. The aralkyl group may comprise up to six carbon atoms, up to eight carbon atoms, up to ten carbon atoms, up to twelve carbon atoms, up to fourteen carbon atoms, up to sixteen carbon atoms, up to eighteen carbon atoms, or up to twenty carbon atoms. If more than one arene ring is present in the aralkyl group, the arene rings may be fused together, or they may be joined by a chemical bond. Arene rings may comprise a heteroatom, for example, nitrogen, oxygen, or sulfur. In compounds of Formula I or pharmaceutically acceptable salts thereof, when both R³ and R⁴ groups are present, and when each of R³ and R⁴ comprises an aralkyl group, R³ and R⁴ may comprise the same aralkyl group, or R³ and R⁴ may comprise different aralkyl groups. Non-limiting examples of aralkyl groups include benzyl, 1-phenylethyl, 2-phenylethyl, 3-phenylpropyl, 2-naphthylethyl, and 9-anthracenylmethyl.

In Formula I, n is an integer from 2 to 5. In some embodiments, the dental composition comprises a compound of Formula I, or a pharmaceutically acceptable salt thereof, wherein n is an integer having a value of about 5, about 4, about 3, or about 2. In some embodiments, n is an integer having a value of 5, or 4, or 3, or 2. It is understood that the dental composition may comprise more than one compound of Formula I, or a pharmaceutically acceptable salt thereof, and that the compounds may be represented by Formula I with different integer values of n. In these embodiments, the average value of n of a composition may be a non-integer.

Preferably, R¹ is a C₁ alkyl group; R³ is a C₈ alkyl group; and n is 4.

It is recognized that the compounds of Formula I include chiral carbon atoms. For simplicity, in Formula I, the stereochemical configuration about each of the chiral carbon atoms is not specified. It is intended that Formula I, as used in this description and in the claims, represents each of the compounds having any of the possible stereochemical configurations. In some embodiments, the compounds of Formula I are amino sugar alcohols and derivatives having the common names D-glucamine, N-methyl-D-glucamine, N-ethyl-D-glucamine, N-octyl-D-glucamine, and N-methyl-N-octanoyl-D-glucamine, N-methyl-N-nonanoyl-D-glucamine, N-methyl-N-decanoyl-D-glucamine. Many of the compounds of Formula I are commercially available. For example, N-methyl-N-nonanoyl-D-glucamine ("MEGA-9") is commercially available from EMD Chemicals, Inc. (San Diego, CA).

Pharmaceutically acceptable salts of compounds of Formula I can also be utilized and can include ammonium salts. In some embodiments, the dental composition of the invention comprises an ammonium salt. An ammonium salt may be represented as the reaction product of an acid with an amine, or as the reaction product of an amine with an alkylating agent such as, for example, iodomethane, bromoethane, or benzyl bromide. An ammonium salt includes a protonated amine compound, for example a compound of Formula I in which a NR¹R² group, wherein R¹ and R² are independently selected from the group consisting of a hydrogen atom and an alkyl group, has been protonated with an inorganic acid or an organic acid. An ammonium salt includes an alkylated amine compound, for example a compound of Formula I in which a NR¹R² group, wherein R¹ and R² are independently selected from the group consisting of a hydrogen atom and an alkyl group, has been alkylated with an alkylating agent.

An ammonium salt comprises at least one counter ion that may be an inorganic anion, an organic anion, or a combination of anions. A combination of anions includes a combination of more than one inorganic anion, a combination of more than one organic anion, or a combination of an inorganic ion and an organic anion. Inorganic ions include, for example, halide (fluoride, chloride, bromide, and iodide), nitrate, sulfate, tetrafluoroborate, and tetra(aryl)borates. Tetra(aryl)borates include compounds having the formula Z₄B⁻, where Z is an aromatic group, for example a substituted or unsubstituted phenyl group. Examples of tetra(aryl)borates include, but are not limited to, tetraphenylborate, tetrakis(4-methylphenyl)borate, tetrakis(2-methylphenyl)borate, tetrakis(1,3,5-trimethylphenyl)borate, tetrakis(4-fluorophenyl)borate, tetrakis(pentafluorophenyl)borate, and tetrakis(4-trifluoromethylphenyl)borate. Organic anions include, for example, alkanoates (such as, for example, acetate, propionate, and butanoate), benzoate, fumarate, maleate, tartrate, ascorbate, benzenesulfonate, toluenesulfonate, and citrate. In some embodiments, the pharmaceutically acceptable salt is free of unsubstituted or substituted tropolone.

In certain implementations, an ammonium salt may be formed by protonation of a compound of Formula I, wherein R¹ and R² are independently selected from a hydrogen atom and an alkyl group, with an inorganic acid, an organic acid, or a combination of an inorganic acid and an organic acid. In another embodiment, an ammonium salt may be formed by alkylation of a compound of Formula I, wherein R¹ and R² are independently selected from a hydrogen atom and an alkyl group, with an alkylating agent. In yet another embodiment, an ammonium salt may be formed by an ion exchange or metathesis reaction with a previously formed ammonium salt. In some embodiments, R¹ comprises an alkyl group and R² is C(O)R³, where R³ comprises an alkyl group. In certain embodiments, R¹ comprises an alkyl group having from about one to about four carbon atoms, and R³ comprises an alkyl group having from about four to about sixteen carbon atoms. In some embodiments, R¹ comprises a methyl group, and R³comprises an alkyl group having seven, eight, or nine carbon atoms. In some embodiments, the dental composition comprises a compound of Formula III, Formula IV, or Formula V.

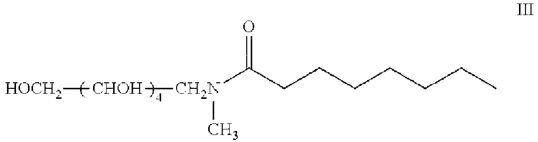

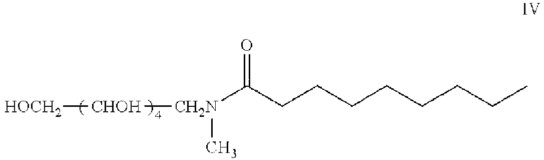

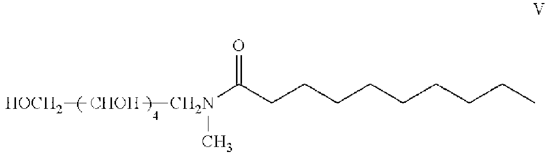

Disclosed compositions include not greater than 3 wt-% of one or more surfactants of Formula I based on the total weight of the composition, not greater than 2.5 wt-% of one or more surfactants of Formula I based on the total weight of the composition, not greater than 2 wt-% of one or more surfactants of Formula I based on the total weight of the composition, or not greater than 1 wt-% of one or more surfactants of Formula I based on the total weight of the composition. Disclosed composition include not less than 0.3 wt-% of one or more surfactants of Formula I based on the total weight of the composition, not less than 0.4 wt-% of one or more surfactants of Formula I based on the total weight of the composition, or not less than 0.5 wt-% of one or more surfactants of Formula I based on the total weight of the composition preferably 0.5 wt-% to 2.5 wt-%.

In some embodiments, disclosed compositions can also include water. The amount of water in a composition can vary greatly depending on the type of composition being formed and the way in which it is to be applied to the oral cavity of the subject. Illustrative types of compositions can include, for example, oral rinses or mouthwashes, toothpastes, gels, oral rinse concentrates, and emulsions specific for xerostomia treatment.

In some embodiments where the composition being formed is an oral rinse or mouthwash, the amount of water can be not less than 60 wt-%, not less than 65 wt-%, not less than 70 wt-%, not less than 75 wt-%, not less than 80 wt%, or not less than 85 wt-% based on the total weight of the composition. In some embodiments where the composition being formed is an oral rinse or mouthwash, the amount of water can be not greater than 98 wt-%, not greater than 97 wt-%, not greater than 95 wt-%, not greater than 93 wt-%, or not greater than 90 wt-% based on the total weight of the composition.

In some embodiments where the composition being formed is a toothpaste, the amount of water can be not less than 5 wt-%, not less than 7 wt-%, not less than 10 wt-%, not less than 15 wt-%, not less than 20 wt%, or not less than 25 wt-% based on the total weight of the composition. In some embodiments where the composition being formed is a toothpaste, the amount of water can be not greater than 65 wt-%, not greater than 60 wt-%, not greater than 55 wt-%, or not greater than 50 wt-% based on the total weight of the composition.

In some embodiments where the composition being formed is a gel, the amount of water can be not less than 10 wt-%, not less than 15 wt-%, not less than 20 wt-%, not less than 30 wt-%, not less than 40 wt%, or not less than 50 wt-% based on the total weight of the composition. In some embodiments where the composition being formed is a gel, the amount of water can be not greater than 95 wt-%, not greater than 90 wt-%, not greater than 85 wt-%, not greater than 80 wt-%, not greater than 75 wt-%, or not greater than 70 wt-% based on the total weight of the composition.

In some embodiments where the composition being formed is a concentrate to make an oral rinse, the amount of water can be not less than 5 wt-%, not less than 7 wt-%, not less than 10 wt-%, not less than 15 wt-%, not less than 20 wt%, or not less than 25 wt- % based on the total weight of the composition. In some embodiments where the composition being formed is a concentrate to make an oral rinse, the amount of water can be not greater than 65 wt-%, not greater than 60 wt-%, not greater than 55 wt-%, or not greater than 50 wt-% based on the total weight of the composition.

In some embodiments where the composition being formed is an emulsion for the treatment of xerostomia, the amount of water can be characterized by the amount of the aqueous phase in the composition. In some embodiments, such a composition can have an amount of aqueous phase of not less than 70 wt-%, not less than 75 wt-%, or not less than 78 wt-% based on the total weight of the composition. In some embodiments where the composition being formed is an emulsion for the treatment of xerostomia, the amount of aqueous phase can be not greater than 95 wt-%, not greater than 92 wt-%, not greater than 90 wt-%, or not greater than 89.5 wt-% based on the total weight of the composition.

Disclosed compositions can also optionally include additional components other than glucosamine or a derivative(s) thereof, and a surfactant of Formula I. Illustrative optional components can include, for example, amino acids, sweeteners, humectants, mineral salts, buffering components, flavorants, preservative agents, tooth re-mineralizing agents, dental caries preventing agents, or combinations thereof. Other optional beneficial ingredients can also be included at appropriate levels such as, folic acid (related to B 12), hyaluronic acid (lubricating, healthy skin), ceramides (healthy skin), arginine, betaines or oxygenated glycerol triesters, vitamin E (antioxidant and preservative), vitamin B12 (healthy skin), EDTA, cetyl pyridinium chloride, chlorhexidine, other antiseptics, or combinations thereof

In some embodiments, disclosed compositions can include amino acids such as glycine, arginine, or combinations thereof. In some embodiments, amounts of glycine, arginine, or combinations thereof in disclosed compositions can be not less than 0.2 wt-%, not less than 0.25 wt-%, not less than 0.5 wt-%, or not less than 0.75 wt-% based on the total weight of the composition. In some embodiments, amounts of glycine, arginine, or combinations thereof in disclosed compositions can be not greater than 3 wt-%, not greater than 2.5 wt-%, or not greater than 2 wt-% based on the total weight of the composition.

In some embodiments, disclosed compositions can include flavorants including for example, peppermint, strawberry, butter, vanilla, coconut, almond, bubble gum, berry, fruit punch, butterscotch, caramel, or combinations thereof. In some embodiments, some flavorants, e.g., mint, citrus, can also be advantageous because they stimulate salivary production when utilized in compositions. Artificial sweeteners may also be used (stevia, aspartame, sucralose, neotame, acesulfame potassium (Ace-K), saccharin, and advantame, for example). In some embodiments, disclosed compositions can include one or more sweeteners including for example, non-cariogenic polyols, or sugar substitutes (e.g., sucralose). In some embodiments, disclosed compositions can include non-cariogenic polyol sweeteners such as xylitol, sorbitol, maltitol, erythritol, isomalt, or combinations thereof. In compositions that include optional sweeteners, the sweetener can be present in an amount that is not less than 2.5 wt-% based on the total weight of the composition or not less than 1 wt-% based on the total weight of the composition. In some embodiments, an optional sweetener can be present in an amount that is not greater than 35 wt-% based on the total weight of the composition or not greater than 20 wt-% based on the total weight of the composition or not greater than 15 wt-% based on the total weight of the composition.

In some embodiments, disclosed compositions can include one or more humectants including for example glycerin, propylene glycol, or sucrose, or combinations thereof. In some embodiments, disclosed compositions can include one or more humectants including for example glycerin, propylene glycol, sucrose or combinations thereof. In compositions that include optional humectants, the one or more humectant can be present in an amount of not less than 2.5 wt-% based on the total weight of the composition, not less than 5 wt- % based on the total weight of the composition, or not less than 10 wt-% based on the total weight of the composition. In compositions that include optional humectants, the one or more humectant can be present in an amount of not greater than 40 wt-% based on the total weight of the composition, not greater than 35 wt-% based on the total weight of the composition, or not greater than 30 wt-% based on the total weight of the composition.

In various embodiments, disclosed compositions can further include one or more tooth re-mineralizing agents alone or in combination with one or more minerals that may be useful or beneficial for ingestion or oral health.

In some embodiments, disclosed compositions can optionally include one or more minerals that may be useful or beneficial for ingestion or oral health. Illustrative optional minerals that can be included in disclosed compositions can include calcium (Ca), phosphorus (P), magnesium (Mg), fluorine (F), iron (Fe), strontium (Sr), zinc (Zn), potassium (K), or combinations thereof. In some embodiments, some minerals can be provided by including magnesium chloride (MgCl₂), calcium chloride (CaCl₂), strontium chloride, zinc chloride, zinc gluconate, potassium nitrate, potassium phosphate dibasic (KH₂PO₄), or combinations thereof. In some embodiments, where fluorine is included, it can be included as the fluoride ion (F-) in salt form (MgF₂, CaF₂), at a concentration that is not greater than 4 milligrams per liter (mg/L).

In particular, some calcium containing compounds are particularly advantageous to include in the disclosed compositions. Such calcium compounds include: calcium chloride, calcium carbonate, calcium caseinate, calcium citrate, calcium glubionate, calcium gluceptate, calcium glycerophosphate, calcium gluconate, calcium hydroxide, calcium hydroxyapatite, calcium lactate, calcium oxalate, calcium oxide lime, calcium pantothenate, dibasic and tribasic calcium phosphate, calcium polycarbophil, calcium propionate, calcium pyrophosphate, calcium silicate, and calcium sulfate. These compounds have been found to minimize demineralization of calcium hydroxyapatite at the surface of the tooth of a patient.

In some embodiments, the tooth re-mineralizing agents (compounds) include phosphate compounds. Suitable phosphate compounds include, but are not limited to, aluminum phosphate, bone phosphate, calcium phosphate, calcium orthophosphate, calcium phosphate dibasic anhydrous, calcium phosphate-bone ash, calcium phosphate dibasic dihydrate, calcium phosphate dibasic anhydrous, calcium phosphate dibasic dihydrate, calcium phosphate tribasic, dibasic calcium phosphate dihydrate, dicalcium phosphate, neutral calcium phosphate, calcium orthophosphate, tricalcium phosphate, precipitated calcium phosphate, tertiary calcium phosphate, whitlockite, magnesium phosphate, potassium phosphate, dibasic potassium phosphate, dipotassium hydrogen orthophosphate, dipotassium monophosphate, dipotassium phosphate, monobasic potassium phosphate, potassium acid phosphate, potassium biphosphate, potassium dihydrogen orthophosphate, potassium hydrogen phosphate, sodium phosphate, anhydrous sodium phosphate, dibasic sodium phosphate, disodium hydrogen orthophosphate, disodium hydrogen orthophosphate dodecahydrate, disodium hydrogen phosphate, disodium phosphate, and mixtures and combinations thereof.

In some embodiments, the calcium compounds, phosphate compounds, or both, are present in the inventive composition in an amount sufficient such that the composition is saturated with at least one of Ca²⁺ ions and PO₄³⁻ ions. In some embodiments, saturated with at least one of calcium ion or phosphate ion can substantially reduce or prevent demineralization, or enhance remineralization, or both, on the surface of the teeth of the patient.

In various embodiments, the tooth re-mineralizing agent is present at up to about 1 part by weight, or up to about 2 parts by weight, or up to about 5 parts by weight.

In some embodiments, disclosed compositions can include one or more preservatives to render the composition microbiologically stable, to increase the microbiological stability thereof, or some combination thereof. In some embodiments, useful preservatives include those that work at a neutral pH, do not detrimentally affect taste, are edible, are effective against a broad spectrum of pathogens, or combinations thereof. Specific illustrative useful preservatives can include GEOGARD^{®} preservatives, which are commercially available from Lonza (Basel, Switzerland) and include benzyl alcohol, sodium benzoate, potassium sorbate, parabens, natural preservatives, polyglyceryl esters, monolaurin,1,2 octanediol, caprylic/capric triglycerides, DHA, aloe vera, potassium sorbate, CPC, PHMB, CHG, vitamin E, triethyl citrate, and EDTA, for example.

In some embodiments, disclosed compositions can be pastes or more specifically toothpastes. Additional optional paste components can include those listed above as well as dental abrasives, carriers, additives, adjuvants, agents, and/or modifiers. Examples of such components include, but are not limited to, an anticaries agent (e.g., a fluoride source), a desensitizing agent, an antigingivitis agent, an antiplaque agent, a rheology modifier, a buffering agent, a diluent, a solvent, a surfactant, a filler, an emulsifier, a foaming agent, a pH modifying agent, a humectant, an antimicrobial agent (e.g., an antibacterial agent), a mouth-feel agent, a texture modifier, an oil, a xerostomia relief agents (e.g., a saliva stimulating agent), an anti-halitosis agent, a breath freshening agents, a medicament, a therapeutic agent, an anti-inflammatory agent, a coloring agent (e.g., a pigment or a dye), a stabilizer, a tartar control agent, carbon black, an anti-erosion agent, a decorative agent, a nutrient, an enzyme (e.g., lysozyme, oxidase), a protein (e.g., enamel matrix protein, proline-rich protein), a wax, other suitable materials, and combinations and mixtures thereof, including for example, those indicated in the categories set forth below. Various dental paste components that can be employed in dental pastes of the present disclosure can also include those disclosed in US Patent No. 5,624,906 (Vermeer).

Disclosed compositions, especially compositions being formed for use as a toothpaste can also include a dental abrasive. In general, the dental abrasive should be suitable for use in an oral environment. The dental abrasive type and characteristics (e.g., particle size, hardness), and the amount of dental abrasive in the dental paste, can be selected so that tooth enamel and dentin are not excessively abraded in the course of normal use of the dental paste, such as during tooth brushing or a during a prophylaxis procedure. The dental abrasive can serve one or more functions including but not limited to abrading and/or otherwise cleaning debris and/or plaque from the tooth surface, removing stains from the tooth surface, polishing the tooth surface, and/or providing a whitening effect on tooth.

Examples of suitable dental abrasives can include, but are not limited to, silica, silica gel, hydrated silica, precipitated silica, fused silica, alumina, calcined alumina, insoluble phosphates, calcium carbonate, ground glass, silicon carbide, ilmenite, sodium bicarbonate, bentonite, mica, zirconia, zirconia silicate, topaz, titanium dioxide, precipitated lime, chalk, pumice (e.g., flour of pumice), zeolites, talcum, kaolin, diatomaceous earth, silicates, glycine, resinous abrasives such as urea-formaldehyde condensation products.

Among phosphates useful as dental abrasives are orthophosphates, polymetaphosphates and pyrophosphates, and salts thereof; illustrative examples are dicalcium orthophosphate dihydrate, dicalcium phosphate, calcium pyrophosphate, tricalcium phosphate, calcium polymetaphosphate, hydroxyapatite, magnesium orthophosphate, and insoluble sodium polymetaphosphate.

Also useful as a dental abrasive of the present disclosure is perlite, for example as described in U.S. Pat. Nos. 5,266,304 (Baffelli et al.) and 6,576,225 (Kilcher et al.).

In some embodiments, a water-soluble dental abrasive can be used (alone or in combination with other dental abrasives, each of which can be water soluble or water insoluble), such as described in U.S. Pat. No. 8,858,921 (Schmid et al.).

In such embodiments, the dental pastes can be substantially free of water (e.g., less than about 5 wt.-% water or less than about 1 wt.- % water, based on the total weight of the dental paste), and optionally include a water miscible or water soluble liquid.

The average particle size of the dental abrasive is generally about 0.1 micrometers, about 1 micrometers, about 5 micrometers, about 10 micrometers, about 15 micrometers, about 20 micrometers, about 25 micrometers, about 30 micrometers, about 35 micrometers, about 40 micrometers, about 50 micrometers, about 75 micrometers, about 90 micrometers, about 100 micrometers, about 150 micrometers, about 200 micrometers, about 250 micrometers, about 300 micrometers, or a range between and including any two of these values. For example, in some embodiments, the average particle size of the dental abrasive is about 0.1 to about 300 micrometers, about 20 to about 300 micrometers, about 90 to about 300 micrometers, about 0.1 to about 50 micrometers, about 1 to about 40 micrometers, or about 5 to about 30 micrometers. As will be appreciated, the dental pastes of the present disclosure can include more than one dental abrasive. In such instances, the average particle size is intended to refer to the average particle size of each dental abrasive included in the dental paste. For example, a dental paste can include, as a first dental abrasive, silica with an average particle size of about 1 to about 40 micrometers and, as a second dental abrasive, calcium carbonate with an average particle size of about 30 to about 75 micrometers.

In certain implementations of the dental pastes of the present disclosure, silica-containing dental abrasives of various types are incorporated because they provide excellent dental cleaning and polishing performance without unduly abrading tooth enamel or dentin. The silica-containing dental abrasive materials described herein, as well as other dental abrasives, generally have an average particle size ranging from about 0.1 to about 30 micrometers or from about 5 to about 15 micrometers. The silica-containing dental abrasive can be precipitated silica or silica gels such as the silica xerogels described in U.S. Pat. Nos. 3,538,230 (Pader et al.) and 3,862,307 (DiGuilio).

Additional types of silica-containing dental abrasives useful in the dental pastes of the present disclosure are described in U.S. Pat. Nos. 4,340,583 (Wason), 5,589,160 (Rice), 5,603,920 (Rice), 5,651,958 (Rice), 5,658,553 (Rice), and 5,716,601 (Rice).

Suitable silica-containing dental abrasives for inclusion into the dental pastes described herein are available from: Huber Engineered Materials under the tradename ZEODENT; Grace under the tradenames SYLOID and SYLODENT; and Evonik under the tradename SIDENT.

One or more dental abrasives are present, in a total amount, of about 4 wt.-%, about 5 wt.-%, about 6 wt.-%, about 8 wt.-%, about 10 wt.-%, about 15 wt.-%, about 20 wt.-%, about 30 wt.-%, about 40 wt.-%, about 50 wt.-%, about 60 wt.-%, about 70 wt.-%, about 80 wt.-%, or a range between and including any two of these values, based on the total weight of the dental paste. In some embodiments, the dental paste includes about 4 wt.-% to about 80 wt.-% dental abrasive(s), based on the total weight of the dental paste. In other embodiments, the dental paste includes about 8 wt.-% to about 50 wt.-%, about 10 wt.-% to about 50 wt.-%, about 10 wt.-% to about 30 wt.-%, or about 5 wt.-% to about 25 wt.-% of the dental abrasive(s) based on the total weight of the dental paste.

Any individual component of a dental paste of the present disclosure can be dissolved, dispersed, suspended, or emulsified in the dental paste. It is understood that while general attributes of each of the categories of components can differ; there can be some common attributes, and any given component can serve multiple purposes within two or more of such categories of components. For example, if sorbitol is included in the dental paste, sorbitol can act a sweetener and a humectant. It is further understood that the components of the dental pastes should be suitable for use (e.g., pharmaceutically acceptable and/or food grade) in the oral environment.

In various embodiments, dental pastes of the present disclosure can include a carrier. The carrier, if present, can include a liquid, a solid, or both. In some embodiments, the carrier can be a liquid at about room temperature. In other embodiments, the carrier can be a solid at about room temperature. In some embodiments, the carrier can be a liquid at about the temperature of the oral cavity of a human, i.e., at about 37 °C. In other embodiments, the carrier can be a solid at about the temperature of the oral cavity of a human. It is understood that a plurality of carriers can be used. Examples of liquid carriers include, but are not limited to, water, glycerin, propylene glycol, polyalkylene glycols (e.g., polyethylene glycol, polypropylene glycol), and combinations thereof.

Further examples of suitable carriers include those described in U.S. Pat. Nos. 6,669,929 (Boyd et al.), 6,379,654 (Gebreselassie et al.), and 4,894,220 (Nabi et al.).

In some embodiments, the carrier (or combinations of carriers) is substantially free of water (e.g., less than about 5 wt.-%, or less than about 1 wt.-% water, based on the total weight of the carrier(s)). In some embodiments where the carrier or combinations of carriers are substantially free of water, such carrier or combination of carriers is water miscible (e.g., water soluble). In further embodiments including carriers (or combinations of carriers) which are substantially free of water, the dental paste is also substantially free of water (e.g., less than about 5 wt.-% water or less than about 1 wt.-% water, based on the total weight of the dental paste). In other embodiments, the dental paste includes water, and water is present in an amount of at least about 10 wt.-%, at least about 20 wt.-%, at least about 30 wt.-%, at least about 40 wt.-%, or at least about 50 wt.-%, based on the total weight of the dental paste.

Each non-carrier component of the dental paste, including but not limited to the compound of Formula I, or pharmaceutically acceptable salt thereof, and the dental abrasive, can independently be dissolved, dispersed, suspended, or emulsified in the carrier. In some embodiments, at least one component of the dental paste is dissolved in the carrier. In some embodiments, at least one component of the dental paste is dispersed in the carrier. In some embodiments, at least one component of the dental paste is suspended in the carrier. In some embodiments, at least one component of the dental paste is emulsified in the carrier. In some embodiments, the compound of Formula I or a pharmaceutically acceptable salt thereof are each dissolved in the carrier.

In some embodiments, dental pastes of the present disclosure can include one or more anticaries agents, such as for example, one or more fluoride sources. Such fluoride sources release, or otherwise provide, fluoride ions in the oral cavity. The fluoride source can be an inorganic fluoride source, an organic fluoride source, or a combination thereof.

Examples of inorganic fluoride sources can include, but are not limited to, alkali metal, alkaline earth metal and ammonium salts of fluoride, such as for example potassium fluoride, sodium fluoride, ammonium bifluoride, calcium fluoride, a copper fluoride (e.g., cuprous fluoride, cupric fluoride), barium fluoride, sodium fluorosilicate, ammonium fluorosilicate, sodium fluorozirconate, sodium monofluorophosphate, aluminum mono- and di-fluorophosphate, fluorinated sodium calcium pyrophosphate, stannous fluoride, zinc fluoride, lithium fluoride, cesium fluoride, aluminum fluoride, indium fluoride, stannous fluorozirconate, ferric fluoride, nickel fluoride, palladium fluoride, silver fluoride, zirconium fluoride, silver diamine fluoride, and combinations thereof. Another class of fluoride sources is fluoride-containing glasses, such as fluoroaluminosilicate glasses. Suitable fluoroaluminosilicate glasses are described in U.S. Pat. Nos. 5,063,257 (Akahane et al.), 4,209,434 (Wilson et al.), and 4,043,327 (Potter et al.) .

In some embodiments, the dental pastes include an inorganic fluoride sources such as sodium fluoride, stannous fluoride, sodium monofluorophosphate, silver diamine fluoride, strontium fluoride, calcium fluoride, fluoroaluminosilicate glass, or a combination thereof.

Organic fluoride sources can include, but are not limited to, N,N,N'-tris(2-hydroxyethyl)-N'-octadecyl-1,3-diaminopropane dihydrofluoride, hexylamine hydrofluoride, laurylamine hydrofluoride, myristylamine hydrofluoride, decanolamine hydrofluoride, octadecenylamine hydrofluoride, myristoxyamine hydrofluoride, diethylaminoethyloctoylamide hydrofluoride, diethanolaminoethyloleylamide hydrofluoride, diethanolaminopropyl-N'-octadecenylamine dihydrofluoride, 1-ethanol-2-hexadecylimidazoline dihydrofluoride, octoylethanolamine hydrofluoride, octyltrimethylammonium fluoride, dodecylethyldimethylammonium fluoride, tetraethylammonium fluoride, dilauryldimethylammonium fluoride, delta 8-9 octadecenylbenzyldimethylammonium fluoride, dioctyldiethylammonium fluoride, cyclohexylcetyldimethylammonium fluoride, furfuryllauryldimethylammonium fluoride, phenoxyethylcetyldimethylammonium fluoride, N,N'-tetramethyl-N,N'-dilaurylethylenediammonium difluoride, N-cetylpyridinium fluoride, N,N-dilaurylmorpholinium fluoride, N-myristyl-N-ethylmorpholinium fluoride, N-(octylaminocarbonylethyl)-N-benzyldimethylammonium fluoride, N-(B-hydroxydodecyl)trimethylammonium fluoride, N-phenyl-N-hexadecyldiethylammonium fluoride, N-cyclohexyl-N-octadecyldimethylammonium fluoride, N-(2-carbomethoxyethyl)-N-benzyldimethylammonium fluoride, N-(2-carbocyclohexoxyethyl)-N-myristyldimethylammonium fluoride, N-(2-carbobenzyloxyethyl)-N-dodecyldimethylammonium fluoride, N-[2-(N,N'-dimethylaminocarbonyl)-ethyl]-N-dodecyidiethylammonium fluoride, N-carboxymethyl-N-cicosyldimethylammonium fluoride, olaflur (N'-octadecyltrimethylendiamine-N,N,N'-tris(2-ethanol) dihydrofluoride), betaine hydrofluoride, sarcosine stannous fluoride, alanine stannous fluoride, glycine potassium fluoride, sarcosine potassium fluoride, glycine hydrofluoride, tetrapropylammonium tetrafluoroborate, tetrabutylammonium tetrafluoroborate, benzyltriethylammonium tetrafluoroborate, lysine hydrofluoride, alanine hydrofluoride, betaine zirconium fluoride, and mixtures thereof. In some embodiments, the organic fluoride source can include tetrapropylammonium tetrafluoroborate, tetrabutylammonium tetrafluoroborate, benzyltriethylammonium tetrafluoroborate, or a combination thereof.

The fluoride source, if present, can be present in the dental pastes in an amount sufficient to release between about 200 ppm to 6,000 ppm fluoride ion, in some embodiments from about 800 to about 1,500 ppm fluoride ion or about 2,500 to about 5,000 ppm fluoride ion. In some dental pastes intended for in-office procedures, the fluoride source is present in an amount sufficient to release between about 10,000 to 23,000 ppm fluoride ion. The fluoride source can be present in the dental paste from about 0.001 wt.-% to about 5 wt.-%, based on the total weight of the dental paste. It is understood that the combination of fluoride sources can be used. The fluoride source can be dissolved, dispersed, suspended, or emulsified in the dental paste. In some embodiments wherein the dental pastes include a carrier, the fluoride source can be dissolved, dispersed, suspended, or emulsified in the carrier.

In some embodiments, dental pastes of the present disclosure can include one or more antibacterial agents. Examples of suitable antibacterial agents can include, but are not limited to, aldehydes (glutaralehyde, phthalaldehyde), salts of phenolics or acids, chlorhexidine or its derivatives (including acid adducts such as acetates, gluconates, chlorides, nitrates, sulfates or carbonates), and combinations thereof.

Non-limiting examples of antibacterial agents include: zinc salts, zinc oxide, tin salts, tin oxide, benzalkonium chloride, hexetidine, long chain alkyl ammonium or pyridinium salts (e.g., cetylpyridinium chloride, tetradecylpyridinium chloride), essential oils (e.g., thymol), furanones, chlorhexidine and salt forms thereof (e.g., chlorhexidine gluconate), sanguinarine, triclosan, stannous chloride, stannous fluoride, octenidine, nonionic or ionic surfactants (e.g., quaternary ammonium compounds), alcohols (monomeric, polymeric, mono-alcohols, poly-alcohols), aromatic alcohols (e.g., phenol)), antimicrobial peptides (e.g., histatins), bacteriocins (e.g., nisin), antibiotics (e.g., tetracycline), aldehydes (e.g., glutaraldehyde) inorganic and organic acids (e.g., benzoic acid, salicylic acid, fatty acids) or their salts, derivatives of such acids such as esters (e.g., p-hydroxybenzoates or other parabens, glycerol esters of fatty acids such as lauricidin), fluoride, EDTA, silver compounds, silver nanoparticles, peroxides (e.g., hydrogen peroxide), and combinations thereof. The antibacterial agent can be dissolved, dispersed, suspended, or emulsified in the dental paste. In some embodiments wherein the dental pastes include a carrier, the antibacterial agent can be dissolved, dispersed, suspended, or emulsified in the carrier. In other embodiments, the dental pastes are free of an antibacterial agent.

Dental pastes of the present disclosure can include one or more inorganic or a natural or synthetic thickeners or gelling agents. Optionally, one or more thickeners are present in a total amount of about 0.01 wt.-% to about 15 wt.-%, in some embodiments about 0.1 wt.-% to about 10 wt.-%, in some embodiments about 0.10 wt.-% to about 5 wt.- %, in some embodiments about 0.2 wt.-% to about 5 wt.-%, and in some embodiments about 0.2 wt.-% to about 1 wt.-%, based on the total weight of the dental paste. In some embodiments, the proportions of thickeners in the dental pastes are sufficient to form an extrudable, shape-retaining product which can be squeezed from a tube onto a toothbrush and will not fall between the bristles of the brush but rather, will substantially maintain its shape thereon. In some embodiments, the thickeners are sufficient to minimize splattering of the dental pastes, if for example a rotating polishing device (e.g., a prophy cup) is used during a polishing or cleaning procedure. In some embodiments, dental pastes of the present disclosure can include at least one thickener, useful for example to impart a desired consistency and/or mouth feel to the dental paste.

Any orally acceptable thickener can be used. Suitable thickeners or gelling agents useful in the dental pastes of the present disclosure include amorphous silica (e.g., as available from Huber Corporation under the trade designation ZEODENT 165), fumed silica, precipitated silica, colloidal silica, natural and synthetic gums and colloids, poloxamers, carbomers, also known as carboxyvinyl polymers, carrageenan, Irish moss, iota-carrageenan, cellulosic polymers such as hydroxyethylcellulose, carboxymethylcellulose (carmellose, cellulose gum) and salts thereof, e.g., carmellose sodium, natural gums such as karaya, xanthan, gum Arabic, gum tragacanth, polyvinylpyrrolidone, agar, colloidal magnesium aluminum silicate, and combinations thereof. One or more thickeners are optionally present in a total amount of about 0.01 wt.- % to about 15 wt.-%, for example about 0.1 wt.-% to about 10 wt.-% or about 0.2 wt.-% to about 5 wt.-%, based on the total weight of the dental paste. The thickener or gelling agent can be dissolved, dispersed, suspended, or emulsified in the dental paste. In some embodiments wherein the dental pastes include a carrier, the thickener or gelling agent can be dissolved, dispersed, suspended, or emulsified in the carrier.

In some embodiments, dental pastes of the present disclosure can include a buffering agent or a buffering system, or a plurality of buffering agents or systems. Examples of suitable buffering agents include phosphate buffers as further described in U.S. Pat. No. 9,149,661 (Pilch et al.).

The buffering agent or buffering system can be dissolved, dispersed, suspended, or emulsified in the dental paste. In some embodiments wherein the dental pastes include a carrier, the buffering agent or buffering system can be dissolved, dispersed, suspended, or emulsified in the carrier.

In some embodiments, dental pastes of the present disclosure can include a coloring agent or a plurality of coloring agents. The coloring agent can be any dye or pigment. The coloring agent can be dissolved, dispersed, suspended, or emulsified in the dental paste. In some embodiments wherein the dental pastes include a carrier, the coloring agent can be dissolved, dispersed, suspended, or emulsified in the carrier.

In some embodiments, dental pastes of the present disclosure can include a desensitizing agent or a plurality of desensitizing agents. The desensitizing agent can serve to combat dentin hypersensitivity. Examples of desensitizing agents include, but are not limited to, a tubule blocking agent, a nerve desensitizing agent, and combinations thereof. Examples of desensitizing agents further include, but are not limited to, strontium salts (e.g., strontium chloride, strontium acetate, and/or strontium nitrate), potassium salts (e.g., potassium citrate, potassium chloride, potassium bicarbonate, potassium gluconate, and/or potassium nitrate), stannous fluoride; basic amino acids in free or salt form (e.g., arginine, lysine, citrulline, ornithine, creatine, histidine, diaminobutanoic acid, and/or diaminopropionic acid, and salts thereof), and combinations thereof. The desensitizing agent can be dissolved, dispersed, suspended, or emulsified in the dental paste. In some embodiments wherein the dental pastes include a carrier, the desensitizing agent can be dissolved, dispersed, suspended, or emulsified in the carrier.

In some embodiments, dental pastes of the present disclosure can include a therapeutic agent or a plurality of therapeutic agents. Such therapeutic agents can be an additive having a therapeutic property. The therapeutic property can include, for example, antiplaque activity, anticaries activity or antimicrobial (e.g., antibacterial) activity. In some embodiments, the therapeutic agent can have biofilm inhibiting or biofilm disrupting activity. The therapeutic agent can be dissolved, dispersed, suspended, or emulsified in the dental paste. In some embodiments wherein the dental pastes include a carrier, the therapeutic agent can be dissolved, dispersed, suspended, or emulsified in the carrier.

In some embodiments, dental pastes of the present disclosure can include a binder or a plurality of binders. The binder can provide a reservoir of the compound of Formula I in an oral cavity of a subject. The compound of Formula I can be released from the binder. The binder can hold the compound of Formula I on or near a surface in an oral cavity of a subject such that, for example, the surface can be exposed to the compound. The surface can be a hard surface, e.g., that which includes a tooth. The surface can be a dental restoration. Examples of suitable binders for are described in U.S. Pat. No. 8,968,709 (Yang et al.) .

The dental pastes can have a form including a solution, a dispersion, a suspension, an emulsion, a solid, a paste, a foam, or a gel. In some embodiments, the dental pastes are toothpastes. In some embodiments, the dental pastes are prophy pastes. Any component of the dental paste can be dissolved, dispersed, suspended, or emulsified in any other component of the dental paste. In some embodiments, one or more of the components are mutually soluble (i.e., miscible with each other).

In some embodiments, the dental paste is provided within a single part or phase. In other embodiments, the dental paste includes both a first and a second part that are separately maintained until they are mixed upon use. Maintaining the first and second parts separately requires only that the parts are maintained in such a way as to substantially prevent the interaction of one part of the dental paste with another part of the dental paste. A dual part dental paste can be employed where there are one or more incompatible ingredients (components) included in the dental paste. For example, if the dental paste includes two incompatible active ingredients, it can be advantageous to maintain them separately. Disclosed compositions can have varied properties. In some embodiments, disclosed compositions can be described by the pH thereof, the viscosity thereof, the stability thereof, various other properties, or combinations thereof.

In some embodiments, disclosed compositions can have a pH that is acceptable for use in the mouth of a person, for example. In some embodiments, disclosed compositions can have a pH from 4.5 to 9.5, for example. In some embodiments, the composition can have a pH in a more neutral range from 5.0-8.5 or 5.5-8.5 for example, as dry mouth sufferers can have a higher sensitivity to pH. The composition can naturally have such a pH or can be buffered to have a pH in a useful, e.g., a "neutral" range.

In some embodiments, disclosed compositions can have a viscosity that renders them useful or deliverable as a sprayable composition. A composition can be sprayable via a non-pressurized dispenser or a pressurized pump dispenser, for example. In some embodiments, disclosed compositions can have a viscosity of not greater than 80,000 centipoise (cps), not greater than 50,000 cps, not greater than 20,000 cps, or not greater than 10,000 cps. In some embodiments, disclosed compositions can have more desirable rheological properties that make them less shear thinning. In some embodiments, less shear thinning compositions have a more widely dispersed spray pattern when delivered in a non-pressurized dispenser.

In some embodiments, disclosed compositions can have desired stability properties. The stability of a composition can include microbiological stability, physical stability, or combinations thereof. In some embodiments, disclosed compositions are microbiologically stable for at least 6 months, in some embodiments 1 year, in some embodiments greater than 2 years.

In some embodiments, disclosed compositions can prevent, inhibit, disrupt the formation or maintenance of a biofilm in an area contacted with the composition. The area contacted can be *in vivo* or *in vitro.* In some embodiments, a composition can prevent, inhibit, disrupt the formation or maintenance of a biofilm in a mouth of a user where the composition was applied to the mouth, for example via spraying the composition into the mouth when compared to a mouth without the composition applied thereto. In some embodiments, a composition can prevent, inhibit, disrupt the formation or maintenance of a biofilm in a container in which a biofilm exists and the composition was applied to the container via pouring, spraying, etc. when compared to a container without the composition applied thereto. Preventing, inhibiting, disrupting, or some combination thereof the formation or maintenance of biofilms can be measured using a modified version of the MBEC assay (described in ASTM E2799-12), which measures disruption of *Streptococcus mutans* biofilms grown on special pegs in a microtiter plate. The biofilms growing on the pegs are treated by periodic submersion into test materials, followed by washing in saliva and water. The biofilm remaining on each peg following treatment is quantified by measuring the amount of fluorescently labeled bacteria that eluted from the pegs at the end of the treatment cycles (see example). In some embodiments, disclosed compositions can affect the buildup of plaque in an area contacted by the composition. The area contacted can be *in vivo* or *in vitro.* In some embodiments, a composition can decrease plaque buildup on at least one tooth in a mouth of a user where the composition was applied to the mouth, for example via spraying the composition into the mouth when compared to a mouth without the composition applied thereto. In some embodiments, a composition can decrease plaque buildup in a container in which plaque can develop and the composition was applied to the container via pouring, spraying, when compared to a container without the composition applied thereto. Decreasing plaque buildup can be measured by a variety of methods *in vivo* including for example plaque scoring, dyeing of plaque.

In another aspect, the present disclosure provides the above composition for use in treating a surface in the oral cavity of a subject, including the steps of (a) providing any one of the dental pastes disclosed herein, and (b) applying the dental paste to a surface in the oral cavity of a subject. The surface in the oral cavity of a subject includes, for example, a buccal surface, a gingival surface, a tooth, a dental restoration, and bone. In some embodiments, the surface is a hard surface, such as for example, the surface of a tooth (including vital surfaces such as enamel and/or dentin, but also non-vital surfaces such as the surface of a dental restoration). The dental pastes can be applied to the oral cavity of a subject by, for example, brushing (e.g., tooth brushing), polishing, swabbing, or combinations thereof. The application of the dental pastes to the surface in the oral cavity can further include polishing the surface, for example polishing the surface of a tooth. While polishing the surface can occur in the course of brushing (e.g., regular tooth brushing at home), polishing can be performed course of a dental prophylaxis procedure, such as that performed in a clinical environment by a dental professional (e.g., a dental hygienist). The subject can be a human, or the subject can be a non-human animal. Non-human animals include mammals such as canines and felines. The dental pastes of the present disclosure can serve to inhibit biofilm formation, for example inhibit biofilm formation on a surface in the oral cavity of a subject. Thus, in another aspect, the present disclosure also provides the above composition for use in treating a surface in the oral cavity of a subject, wherein the composition is to be administered in a method including the steps of (a) providing any one of the dental pastes disclosed herein, and (b) applying the dental paste to a surface in the oral cavity of a subject. The surface in the oral cavity of the subject and the manner of application of the dental paste can be any of those previously described.

In some embodiments, disclosed compositions can affect hydration loss in an area contacted by the composition. The area contacted can be *in vivo* or *in vitro.* In some embodiments, a composition can decrease hydration loss in a mouth of a user where the composition was applied to the mouth, for example via spraying the composition into the mouth when compared to a mouth without the composition applied thereto. In some embodiments, a composition can decrease hydration loss from a tissue in which hydration can be lost and the composition was applied to the tissue via pouring, spraying, when compared to a tissue without the composition applied thereto. Hydration loss can be measured by exposing treated tissues of uniform size to a controlled 37°C environment for a set time period (4 hrs), and recording the % weight loss from the treated tissue sample. The treated tissue is then exposed to high temperature to rid the sample of all water (95°C / 4 hrs and 115°C / 4 hrs). The water lost at 4 hrs is divided by the total water loss (after the 115°C step) and is indicative of the water lost from the tissue at 4 hrs. In some embodiments, disclosed compositions can affect less than 65% water loss, or less than 60% water loss.

In some embodiments, disclosed compositions can affect lubricity or lubriciousness of an area contacted by the composition. The area contacted can be *in vivo* or *in vitro.* In some embodiments, a composition can maintain or increase lubricity in a mouth of a user where the composition was applied to the mouth, for example via spraying the composition into the mouth when compared to a mouth without the composition applied thereto. In some embodiments, a composition can provide lubricating properties to a mouth to the same degree that saliva can, for example. In some embodiments, a composition can maintain or increase lubricity on a substrate in which lubricity can be lost and the composition was applied to the substrate via pouring, spraying, etc. when compared to a substrate without the composition applied thereto. Lubricity or the ability to provide lubricating properties can be measured by the friction coefficient relative to a suitable substrate. A low friction coefficient (comparable to saliva) is desired.

Also disclosed herein are the disclosed compositions for use in contacting an oral cavity or oral tissue with the composition. The step of contacting the oral cavity or oral tissue can be accomplished by applying the composition in any way, for example spraying. Disclosed compositions can be useful for preventing, inhibiting, disrupting, or any combination thereof the formation or maintenance of a biofilm in an area contacted with the composition; for affecting hydration loss in an area contacted by the composition; for affecting lubricity or lubriciousness of an area contacted by the composition.

All scientific and technical terms used herein have meanings commonly used in the art unless otherwise specified. The definitions provided herein are to facilitate understanding of certain terms used frequently herein and are not meant to limit the scope of the present disclosure.

The term "paste" refers to a substance that behaves as a solid until a sufficiently large load or stress is applied, at which point it flows like a liquid. Pastes can include a suspension of a solid in a background fluid or carrier. Pastes can be classified by their viscosity or their consistency.

The term "dental paste" refers to a paste that is used in the oral cavity of a subject.

The term "toothpaste" refers to a type of dental paste used as a cleaning agent for regular individual care, such as through daily tooth brushing. Toothpastes can be used as a prophylactic measure against caries, gingivitis, or periodontitis.

The term "prophylaxis paste" (or more simply, "prophy paste") refers to a type of a dental paste used by a dental professional, such as a dentist or dental hygienist, to remove adherent deposits such as stain, plaque, or tartar which can stick to a hard surface in the oral cavity of a subject. Such adherent deposits may not be fully removed in the course of regular tooth brushing. A prophy paste can be used on a rotating prophy paste holder referred to as a "prophylaxis cup" (or more simply, "prophy cup"). Prophy pastes can also be applied with a brush, such as a rotating brush. Commercially available prophy pastes can have a different viscosity in comparison to commercially available toothpastes.

The term "therapeutic" refers to preventing, ameliorating, treating, improving, or curing a disease or condition.

The term "biofilm" refers to a matrix including bacteria. Along with bacteria, a biofilm in the oral cavity of a subject can further include epithelial cells, leukocytes, macrophages, and other oral exudate.

The term "biofilm inhibiting" refers to limiting the formation or growth of a biofilm.

The term "hard surface" refers to a surface in the oral cavity of a subject, including hard material, such as bone, dental enamel, dentin, and dental restorations.

The term "dental restorations" refers to fillings, inlays, onlays, veneers, temporary and permanent crowns or bridges, implants, or orthodontic devices and appliances such as brackets or archwires.

The term "alkyl" refers to a monovalent group that is a radical of an alkane, which is a saturated hydrocarbon. The alkyl group can be linear, branched, cyclic, or combinations thereof and can have 1 to 20 carbon atoms. **In** some embodiments, the alkyl group includes 1 to 18, 1 to 12, 1 to 10, 1 to 8, 1 to 6, or 1 to 4 carbon atoms. Examples of alkyl groups include, but are not limited to, methyl, ethyl, n-propyl, isopropyl, n-butyl, isobutyl, tert-butyl, n-pentyl, n-hexyl, cyclohexyl, n-heptyl, n-octyl, and ethylhexyl.

The terms "room temperature" and "ambient temperature" are used interchangeably to mean a temperature in the range of 20 °C to 25 °C.

As used in this specification and the appended claims, the singular forms "a", "an", and "the" encompass embodiments having plural referents, unless the content clearly dictates otherwise.

As used in this specification and the appended claims, the term "or" is generally employed in its sense including "and/or" unless the content clearly dictates otherwise. The term "and/or" means one or all of the listed elements or a combination of any two or more of the listed elements.

As used herein, "have", "having", "include", "including", "comprise", "comprising" or the like are used in their open ended sense, and generally mean "including, but not limited to". It will be understood that "consisting essentially of", "consisting of", and the like are subsumed in "comprising" and the like. For example, a composition that "comprises" silver may be a composition that "consists of" silver or that "consists essentially of" silver.

As used herein, "consisting essentially of," as it relates to a composition, apparatus, system, method or the like, means that the components of the composition, apparatus, system, method or the like are limited to the enumerated components and any other components that do not materially affect the basic and novel characteristic(s) of the composition, apparatus, system, method or the like.

The words "preferred" and "preferably" refer to embodiments that may afford certain benefits, under certain circumstances. However, other embodiments may also be preferred, under the same or other circumstances. Furthermore, the recitation of one or more preferred embodiments does not imply that other embodiments are not useful, and is not intended to exclude other embodiments from the scope of the disclosure, including the claims.

Also herein, the recitations of numerical ranges by endpoints include all numbers subsumed within that range (e.g., 1 to 5 includes 1, 1.5, 2, 2.75, 3, 3.80, 4, 5, or 10 or less includes 10, 9.4, 7.6, 5, 4.3, 2.9, 1.62, 0.3)

Where a range of values is "up to" a particular value, that value is included within the range.

Use of "first," "second," in the description above and the claims that follow is not intended to necessarily indicate that the enumerated number of objects is present. For example, a "second" substrate is merely intended to differentiate from another substrate (such as a "first" substrate). Use of "first," "second," in the description above and the claims that follow is also not necessarily intended to indicate that one comes earlier in time than the other.

Example articles and techniques according to the disclosure provide will be illustrated by the following non-limiting examples.

### EXAMPLES

**Table 1. Materials**

| **Material** | **CAS** | **Manufacturer** |
|---|---|---|
| Deionized Water (DIW) | n/a | In-house MILLIPORE Milli-Q Water System |
| N-Nonanoyl-N-methylglucamine (MEGA-9) | 85261-19-4 | 3M Corp., St. Paul, MN |
| Glucosamine HCl | 66-84-2 | Sigma-Aldrich, St. Louis, MO |
| Sorbitol (Spectrum) (70 wt.-% aqueous solution) | 50-70-4 | Spectrum Inc., New Brunswick, NJ |
| Sorbitol (Sigma) (70 wt.-% aqueous solution) | 50-70-4 | Sigma Aldrich; St. Louis, MO, USA |
| Glycerol (Sigma) | 56-81-5 | Sigma-Aldrich, St. Louis, MO |
| Glycerol (Spectrum) | 56-81-5 | Spectrum Chemical; New Brunswick, NJ, USA |
| Titanium dioxide (Type MT-500B) | 13463-67-7 | Diacolor-Pope Inc., Paterson, NJ |
| carboxymethyl cellulose (CMC) | 9004-32-4 | Ashland Inc., Covington, KY |
| Silica abrasive | 7631-86-9 | Evonik Industries AG, Essen, Germany |
| Silica thickener | 7631-86-9 | J.M. Huber Corp., Edison, NJ |
| Sodium fluoride | 7681-49-4 | Sigma-Aldrich, St. Louis, MO |
| Sodium saccharin | 128-44-9 | Sigma-Aldrich, St. Louis, MO |
| Sucralose | 56038-13-2 | Sigma-Aldrich, St. Louis, MO, USA |
| Hydroxyethyl Cellulose | 9004-62-0 | Ashland Inc., Covington, KY |
| Cetylpyridinium Chloride | 6004-24-6 | TCI America; Portland, OR, USA |
| K₂HPO₄ | 16788-57-1 | MP Biomedicals; Aurora, OH, USA |
| KH₂PO₄ | 7778-77-0 | VWR; West Chester, PA, USA |
| Xylitol | 87-99-0 | Roquette Pharma; Geneva, IL, USA |
| Erythritol | 149-32-6 | Cargill; Wayzata, MN USA |
| Glycine | 56-40-6 | 3M Company; St. Paul, MN, USA |
| Arginine | 32042-43-6 | TCI America, Portland, Oregon. |
| Spearmint Flavor | N/A | Symrise, Teterboro, NJ |
| PERIDEX Oral Rinse (0.12 wt% chlorhexidine gluconate) | n/a | 3M Corp., St. Paul, MN |
| COLGATE TOTAL Clean Mint Anticavity Fluoride and Antigingivitis toothpaste | n/a | Colgate-Palmolive Co., New York, NY |

As used herein, all parts and percentages are by weight, all water is deionized water ("DI water"), and all molecular weights are average molecular weights, unless otherwise specified.

### MBEC Biofilm Disruption Assay Procedure:

This assay, which utilizes the high throughput MBEC assay system (Innovotech, Calgary, AB Canada), evaluates efficacy of a treatment in disrupting biofilm by quantifying the amount of fluorescent-labeled biofilm remaining on MBEC inoculator pegs after exposure to relevant treatment conditions. The assay is similar to ASTM E2799-12 *(Standard Method for Testing Disinfectant Efficacy against Pseudomonas aeruginosa Biofilm using the MBEC*^{™} *Assay)* but was modified for use with *Streptococcus mutans* as the relevant organism and used a modified protocol for exposure of the biofilms to test materials.

An overnight culture of *Streptococcus mutans* (ATCC 25175) was prepared by using a sterile inoculation loop to introduce a small amount of frozen stock into 5 milliliter of brain heart infusion (BHI) broth in a 15-mL conical tube. The culture was grown at 37°C (static, not shaking) for 12-16 hours. The density of the overnight culture is estimated by turbidity measurement (OD600), and diluted appropriately to OD600 = 0.01 in BHI media supplemented with 1% Sucrose and 1 micromolar ALEXA FLUOR 488 dextran - 10,000 MW (Molecular Probes^{®} PN/D22910) to obtain an appropriate volume of inoculum to fill the required 96-well plate. 150 microliter of inoculum is added to the appropriate wells of a 96 well MBEC Biofilm Inoculator plate (Innovotech). Control wells for fluorescence readings were also filled with 150 microliter of phosphate-buffered saline (PBS) or non-bacteria-containing media consisting of BHI with 1% sucrose and 1 micromolar ALEXA FLUOR 488 dextran. The MBEC inoculator lid (with pegs positioned to be submerged in each well) is placed over the inoculated plate. The plate is wrapped in parafilm and incubated in a sealed plastic container humidified by lining the bottom of the plastic container with a wet paper towel (37⁰C, shaking at 250 RPM). Biofilm was allowed to form on the pegs for 4 hours prior to exposure of the biofilms to example formulations. In this modified assay, the biofilms growing on the pegs were exposed the example formulations 3 times during the assay, at 4, 7 and 24 hours post inoculum. The exposure to example formulations was performed as a treatment cycle with additional washing steps in artificial saliva and water. Treatment occurred by transferring the pegs into various 96-well plates filled with 150 microliter per well of example formulations or 150 microliter per well of artificial saliva or water.

The treatment cycle steps (performed at 4, 7, and 24 hours) were:
1) 1-minute exposure to test materials (150 microliter/well) - 37°C -shaking)
2) 1-minute exposure to sterile water (200 microliter/well) 37°C, shaking)

After the water rinse, the pegs were returned to a 96 well plate filled with growth media (BHI with 1% Sucrose and 1 micromolar ALEXA FLUOR 488 dextran - 10,000 MW) and returned to the incubator to allow for additional biofilm growth until the next treatment or until the end of the final growth period. Following the last treatment exposure cycle (at 24 hours post inoculation), the pegs were incubated in growth media for an additional 4 hours before transferring the MBEC pegs into a final wash plate for 1 minute (containing 200 microliter sterile water per well, shaking at 37°C for final rinsing), and then into a 96 well black plate (suitable for fluorescence quantification in a plate reader) filled with 210 uL/well of 50 U/milliliter dextranase (from Penicillum sp., SIGMA D4668-1KU) in 0.2M Acetate, pH 5.5. The pegs were incubated in the extraction solution wells by shaking at 250 RPM at 37°C for 30 minutes. The plate was then placed in a sonicating water bath for at least 1 hour at room temperature to elute the accumulated biofilm from the pegs into the Dextranase/Acetate solution. Following sonication, the fluorescence in each well is read in a fluorescence plate reader (excitation = 488 nm, emission =525 nm). Replicates of 4-8 wells per treatment were performed.

### MBEC Biofilm Disruption Assay Results

Table 2 shows the average relative fluorescence reading for each example formulation treatment. Low values signify greater biofilm disruption activity than high values. PERIDEX was used as a positive treatment control and is expected to show no biofilm accumulation. Glucosamine HCl enhanced the biofilm disruption effect of MEGA-9, and in some instances, the effect is synergistic.

**Table 2. Comparative Examples C1-C13 and Examples Ex.1- Ex.4, Formulations (percentage by weight) and MBEC Biofilm Test Data**

| Example | DIW | MEGA-9 | Glucosamine HCl | Fluorescenc e Reading | Standard Deviation |
|---|---|---|---|---|---|
| C1 | 100% | N/A | N/A | 3842 2 | 671 9 |
| C2 | 99.75 % | 0.25 % | N/A | 3811 9 | 535 7 |
| C3 | 99.5% | 0.5% | N/A | 2881 3 | 536 |
| C4 | 99.25 % | 0.75 % | N/A | 347 | 8 |
| C5 | 99.75 % | N/A | 0.25 % | 4202 1 | 240 5 |
| C6 | 99.25 % | N/A | 0.75 % | 4228 9 | 205 |
| C7 | 98% | N/A | 2% | 3947 4 | 425 4 |
| C8 | 99.5% | 0.25 % | 0.25 % | 4207 3 | 484 4 |
| C9 | 99% | 0.25 % | 0.75 % | 4282 7 | 181 8 |
| C1 0 | 97.75 % | 0.25 % | 2% | 4154 5 | 447 8 |
| C1 1 | 99.25 % | 0.5% | 0.25 % | 2542 2 | 885 4 |
| C1 2 | 98.75 % | 0.5% | 0.75 % | 1082 9 | 917 5 |
| Ex. 1 | 97.5% | 0.5% | 2% | 494 | 190 |
| Ex. 2 | 99% | 0.75 % | 0.25 % | 269 | 54 |
| Ex. 3 | 98.5% | 0.75 % | 0.75 % | 216 | 36 |
| Ex. 4 | 97.25 % | 0.75 % | 2% | 206 | 48 |
| C1 3 | PERIDEX | | | 419 | 153 |

### Microcosm Biofilm Disruption Test - Procedure

A 1-L stock solution of mucin-containing medium (MCM) supplemented with ~5 wt.-% sucrose was prepared as follows. 1 g Lab-Lemco beef extract powder (Thermo Fisher Scientific, Waltham, MA), 2 g yeast extract powder (Becton, Dickinson and Company, Franklin Lakes, NJ), 5 g proteose peptone (Becton, Dickinson and Company, Franklin Lakes, NJ), 2.5 g Type III porcine stomach mucin (Sigma-Aldrich, St. Louis, MO), 0.35 g sodium chloride, 0.2 g calcium chloride dihydrate, and 0.2 g potassium chloride were dissolved in 800 milliliter of deionized water. The pH was measured with a calibrated pH electrode (pH = 6.9). An additional 100 milliliter deionized water was added and the resulting solution autoclaved 15 minutes at 121°C . After cooling to room temperature, 1.25 milliliter of filter sterilized 40 w/v% aqueous urea was added, followed by 98.75 milliliter of autoclaved 50 w/v% aqueous sucrose.

Composite disks (10 mm diameter x 2.1 mm thickness) sectioned from a commercially available dental mill blank (PARADIGM MZ100, available from 3M Corp., St. Paul, MN) and polished to a final thickness of 2.0 mm with a 1000 grit finish were used as substrates for biofilm growth. Prior to use, the disks were disinfected in 75 v/v% ethanol for 45 to 60 minutes. Disks were cleaned for reuse by shaking them in 1% aqueous sodium hydroxide to remove biofilm growth, rinsed with water, and stored in 50% aqueous ethanol.

One sterilized composite disk was placed in each well of a sterile 24-well plate. Human saliva from a single healthy donor was mixed with MCM at saliva:MCM=1:4; 1.8 milliliter of this mixture was added to each disk-containing well. The 24-well plate was placed in an incubator at 37 °C with shaking (60 rpm) for 4 hours. The plate was removed from the incubator, the growth medium was removed, 1.8 milliliter fresh MCM (no saliva) was added to each well, the plate was then incubated 4 hours at 37 °C with shaking. Hereafter, only MCM without saliva was used for further medium exchange.

The disks were then subjected to five treatment/growth cycles as follows. Liquid was removed from the wells via pipette. 1.8 milliliter of treatment solution was added to each well, and then removed after 1 minute of disk exposure. Each disk was then rinsed with deionized water for 2 min, 1.8 milliliter MCM added to each well, and the plate incubated for 4 hours at 37 °C. For overnight storage under refrigeration, the MCM was removed and replaced with phosphate-buffered saline (PBS; also known as "Dulbecco A", described by Dulbecco & Vogt 1954, J. Exp. Med. 99, 167-182). The last medium change was performed two hours before end of the test method.

After the last treatment/growth cycle on the fourth day, the biofilms were collected with dried filter paper to determine the anhydrous biofilm mass for each disk. The disks with the grown biofilm were carefully transferred into new 24-well-plates filled with 1.0 milliliter deionized water per well. Each disk was washed with 1.0 milliliter deionized water three times for 1 min with moderate shaking (ca. 400 rpm). After the washing, the disks were carefully blotted on filter paper to remove excess liquid. The biofilms were then removed from the disks by wiping the surfaces of each disk with filter paper which had been dried overnight in a microcentrifuge tube at 70°C and weighed to determine the total weight of the dried paper and the microcentrifuge tube. Each filter paper with the wet biofilm was then placed back into the same microcentrifuge tube, dried overnight at 70°C, and biofilm mass determined by weighing the dry biofilm, paper, and microcentrifuge tube, and subtracting the initial mass of the tube and paper. The steps of the Microcosm Biofilm Disruption Test are summarized in the Table 3.

**Table 3.**

| | |
|---|---|
| Day 1 | Sterilize substrate disks, place into wells |
| | Disk inoculation with human saliva / MCM mixture (4 hours at 37 °C) |
| | Remove human saliva / MCM mixture |
| | Add MCM (4 hour growth phase at 37 °C) |
| | Remove MCM and replace with PBS |
| | Refrigerate overnight |
| Days 2 and 3 | Remove PBS |
| | Add treatment solution, expose disk for 1 min, remove treatment solution, rinse disk with DI water |
| | Add MCM (4 hour growth phase at 37 °C) |
| | Remove MCM |
| | Add test formulation, expose disk for 1 min, remove treatment solution, rinse disk with DI water |
| | Add MCM (4 hour growth phase at 37 °C) |
| | Remove MCM and replace with PBS |
| | Refrigerate overnight |
| Day 4 | Remove PBS |
| | Add treatment solution, expose disk for 1 min, remove treatment solution, rinse disk with DI water |
| | Add MCM (4 hour growth phase at 37 °C) |
| | Collect biofilms and dry overnight |
| Day 5 | Weigh dried biofilms |

**Table 4. Toothpaste Formulations (percentage by weight)**

| Ingredient | Comparative Example C14 (base toothpaste) | Comparative Example C15 (base + 2% MEGA-9) | Comparative Example C16 (base + 2% glucosamine) | Example Ex.5 (base + 2% MEGA-9, 2% glucosamine |
|---|---|---|---|---|
| Glycerol (Sigma) | 8.55 | 8.55 | 8.55 | 8.55 |
| CMC | 1.00 | 1.00 | 1.00 | 1.00 |
| DIW | 43.44 | 41.44 | 41.44 | 39.44 |
| Titanium dioxide | 0.70 | 0.70 | 0.70 | 0.70 |
| glucosamine | 0.00 | 0.00 | 2.00 | 2.00 |
| MEGA-9 | 0.00 | 2.00 | 0.00 | 2.00 |
| Sorbitol (Spectrum) | 25.38 | 25.38 | 25.38 | 25.38 |
| Sodium fluoride | 0.21 | 0.21 | 0.21 | 0.21 |
| Sodium saccharin | 0.27 | 0.27 | 0.27 | 0.27 |
| Silica thickener | 6.50 | 6.50 | 6.50 | 6.50 |
| Silica abrasive | 13.95 | 13.95 | 13.95 | 13.95 |

To prepare test (treatment) solutions for use in the Biofilm Microcosm Test Method, each of the toothpaste formulations in Table 4 were mixed with DI water (toothpaste:DI water = 1:3, by weight), the resultant mixtures centrifuged, and the supernatant aqueous layers from the centrifuged mixtures were removed and used as the test solutions. In the same fashion, a test solution was prepared from a commercially available toothpaste (Colgate Total Clean Mint, Anticavity Fluoride and Antigingivitis toothpaste, including 0.30% triclosan, "CTOT") by mixing with DI water (toothpaste: DI water = 1:3, by weight), centrifuging the mixture, and recovering the supernatant for testing.

**Table 5. Microcosm Biofilm Test Data. Groups with the same letter are statistically not different (SND) via One-Way ANOVA with Tukey's t-test. StDev= standard deviation; N= number of replicates.**

| | DIW | CTOT | Comparative Example C14 (base toothpaste) | Comparative Example C15 (base + 2% MEGA-9) | Comparative Example C16 (base + 2% glucosamine) | Example Ex.5 (base + 2% MEGA-9, 2% glucosamine) |
|---|---|---|---|---|---|---|
| Mean Biomass (mg) | 1.65 | 0.08 | 1.50 | 1.42 | 1.34 | 0.65 |
| StDev | 0.40 | 0.34 | 0.23 | 0.25 | 0.31 | 0.39 |
| N | 18 | 18 | 18 | 18 | 18 | 18 |
| SND groups | A | C | A | A | A | B |

The data in Table 5 show that toothpastes including only MEGA-9 (C15) or only glucosamine (C16) display little inhibitory effect on biofilm growth, resulting in biomass amount similar to the negative control (DI water) and the base toothpaste (C14). In contrast, a toothpaste including both MEGA-9 and glucosamine (Ex.5) provides significant inhibition in biofilm growth, similar to that of the commercially available antibacterial-containing toothpaste COLGATE TOTAL (CTOT).

### Example Ex.6: Oral Care Aqueous Solution

An aqueous formulation is shown in Table 6. It can be used as oral rinse, spray, lozenge filling, for treatment of xerostomia, halitosis.

Other additives may be incorporated for e.g. anticaries, antiplaque, antigingivitis, antisensitivity.

**Table 6. Aqueous Oral Formulation Example**

| **Ingredient** | **Weight %** |
|---|---|
| MEGA-9 | 0.4% |
| Glucosamine | 1.0% |
| Glycerol (Spectrum) | 15.6% |
| sucralose | 0.0857% |
| Hydroxyethyl Cellulose | 0.05% |
| Cetylpyridinium Chloride | 0.05% |
| K₂HPO₄ | 1.0712% |
| KH₂PO₄ | 0.5239% |
| Xylitol | 1.0% |
| Sorbitol (Sigma) | 1.0% |
| Erythritol | 1.0% |
| Glycine | 1.0% |
| Arginine | 1.0% |
| Spearmint Flavor | 0.2% |
| DI water | 76% |

### Friction Coefficient Procedure

Friction coefficient was measured using a FORCEBOARD universal friction tester (Industrial Dynamics, Sweden AB) affixed with a 10 newton (N) load cell. A natural lambskin condom made from sheep cecum (Trojan NATURALAMB^{™} Luxury Condoms, Church & Dwight Co., Inc. Ewing, NJ) was used to mimic oral mucosal tissue. The condom was rinsed thoroughly and excess liquid was removed prior to use and between measurements to assure that no residual lubricant or test formulation interfered with subsequent measurements. The condom was placed on a glass slide, smoothed to remove wrinkles, and clamped to the FORCEBOARD friction tester using a binder clip. A syringe was used to meter 0.5 milliliter of test solution onto the surface of the condom near the front of the FORCEBOARD (farthest from the motor). A second layer of condom was then secured around a gloved finger, and the covered finger was moved down the surface of the FORCEBOARD (toward the motor) while applying a target vertical force of 3 N. This motion was repeated 6 times. Friction coefficients (at a vertical force of 2.9 to 3.1 N) were calculated by the FORCEBOARD Analyzer software (Industrial Dynamics, Sweden).

Results for friction coefficient are shown in Table 7. A low friction coefficient value is more slippery and thus is a desirable property for a formulation to treat xerostomia. The friction coefficient of Example 19 is less than that of water.

**Table 7. Friction coefficient results.**

| | Mean Friction Coefficient | StDev | N |
|---|---|---|---|
| Deionized Water | 0.39 | 0.05 | 3 |
| Example Ex.6 | 0.27 | 0.03 | 3 |

Thus, embodiments of oral compositions and their use are disclosed. The implementations described above, and other implementations are within the scope of the following claims. One skilled in the art will appreciate that the present disclosure can be practiced with embodiments other than those disclosed as long as the embodiment is within the scope of the claims. The disclosed embodiments are presented for purposes of illustration and not limitation.

## Claims

1. A composition comprising:
from 1 wt-% to 3 wt-% total of glucosamine, a derivative of glucosamine, or a combination thereof based on the total weight of the composition, wherein the derivative of glucosamine is selected from salts of glucosamine and N-acetylglucosamine; and
from 0.3 wt-% to 3 wt-% total of one or more surfactants, based on the total weight of the composition, of Formula I:
HOCH₂-(CHOH)ₙ-CH₂NR¹R² (1)
wherein
R¹ and R² are independently selected from the group consisting of a hydrogen atom, an alkyl group, C(O)R³, and SO₂R⁴;
with R³ and R⁴ being independently selected from the group consisting of an alkyl group, an aryl group, and an aralkyl group; and
n is an integer from 2 to 5,
with the proviso that the following Aqueous Solution A is excluded, wherein DMSO is dimethyl sulfoxide:
Aqueous Solution A
| Ingredient | Wt% |
|---|---|
| Water | 80.10 |
| Glycerin | 10.00 |
| MEGA-9 | 0.70 |
| Octyl Glucopyranoside | 0.70 |
| N-Acetyl-D-Glucosamine | 0.50 |
| N-Acetyl-D-Galactosamine | 0.50 |
| D-Glucosamine Hydrochloride | 0.50 |
| D-Glucosamine Sulfate | 0.50 |
| D-Glucamine | 0.50 |
| N-Methyl-D-Glucamine | 0.50 |
| N-Ethyl-D-Glucamine | 0.50 |
| N-Octyl-D-Glucamine | 0.50 |
| Xylitol | 0.50 |
| Sorbitol | 0.50 |
| Erythritol | 0.50 |
| Mannitol | 0.50 |
| Glycine | 0.50 |
| Arginine | 0.50 |
| Nonanoic Acid | 0.50 |
| Ethyl Caprylate | 0.50 |
| DMSO | 0.50 |
| TOTAL | 100.00 |

2. The composition of claim 1, wherein R¹ is a hydrogen atom or a C₁-C₂ alkyl group.

3. The composition of claim 1, wherein R³ is a C₇-C₉ alkyl group, preferably a C₈ alkyl group.

4. The composition according to claim 1, wherein the total of glucosamine, a derivative of glucosamine, or a combination thereof based on the total weight of the composition is from 1.5 wt-% to 2.5 wt%.

5. The composition according to claim 1, wherein the total of one or more surfactants, based on the total weight of the composition, of Formula I is from 0.5 wt-% to 3 wt-%.

6. The composition of any of claims 1 to 5 further comprising water in an amount from 5 wt-% to 98 wt-% based on the total weight of the composition.

7. The composition of claim 6, wherein the composition is a gel and the composition has from 10 wt-% to 95 wt-% water based on the total weight of the composition.

8. The composition of claim 6, wherein the composition is a rinse concentrate and the composition has from 5 wt-% to 65 wt-% water.

9. The composition of claim 6, wherein the composition is an oral rinse and the composition is an emulsion having an aqueous and an oil phase.

10. The composition of claim 1, wherein the composition is a toothpaste and further comprises one or more dental abrasives.

11. The composition according to claim 1, further comprising arginine, glycine, or a combination thereof in an amount from 0.2 wt-% to 3 wt-% based on the total weight of the composition.

12. The composition of any one of claims 1 to 11 for use in preventing, inhibiting, disrupting, or any combination thereof the formation or maintenance of a biofilm in an oral tissue, wherein the composition is to be administered in a method comprising:
contacting an oral tissue with a composition according to any of claims 1 to 11, wherein the proviso of claim 1 does not apply.

13. The composition of any one of claims 1 to 11 for use in treating a surface in the oral cavity of a subject, wherein the composition is to be administered in a method comprising:
(a) providing the composition of any one of claims 1 to 11; and
(b) applying the composition to a surface in the oral cavity of the subject, wherein the proviso of claim 1 does not apply.

14. The composition for use of claim 12 or 13 for use in the treatment or prevention of dental plaque, dental caries, gingivitis, and/or periodontal diseases.

15. A method of preventing, inhibiting, disrupting, or any combination thereof the formation or maintenance of a biofilm, wherein the method comprises *in vitro* contacting an area with the composition of any one of claims 1 to 11, wherein the proviso of claim 1 does not apply.

## Patentansprüche

1. Eine Zusammensetzung, umfassend:
von 1 Gew.-% bis 3 Gew.-% Gesamtanteil von Glucosamin, einem Derivat von Glucosamin oder einer Kombination davon, bezogen auf das Gesamtgewicht der Zusammensetzung, wobei das Derivat von Glucosamin aus Salzen von Glucosamin und N-Acetylglucosamin ausgewählt ist; und
von 0,3 Gew.-% bis 3 Gew.-% Gesamtanteil von einen oder mehreren Tensiden der Formel I, bezogen auf das Gesamtgewicht der Zusammensetzung: HOCH₂-(CHOH)ₙ-CH₂NR¹R² (I) wobei
R¹ und R² unabhängig voneinander aus der Gruppe bestehend aus einem Wasserstoffatom, einer Alkylgruppe, C(O)R³ und SO₂R⁴ ausgewählt sind;
wobei R³ und R⁴ unabhängig voneinander aus der Gruppe bestehend aus einer Alkylgruppe, einer Arylgruppe und einer Aralkylgruppe ausgewählt sind; und
n eine ganze Zahl von 2 bis 5 ist,
mit der Maßgabe, dass die folgende wässrige Lösung A ausgeschlossen ist, wobei DMSO Dimethylsulfoxid ist:
Wässrige Lösung A
| Bestandteil | Gew.-% |
|---|---|
| Wasser | 80,10 |
| Glyzerin | 10,00 |
| MEGA-9 | 0,70 |
| Octylglucopyranosid | 0,70 |
| N-Acetyl-D-Glucosamin | 0,50 |
| N-Acetyl-D-Galactosamin | 0,50 |
| D-Glucosaminhydrochlorid | 0,50 |
| D-Glucosaminsulfat | 0,50 |
| D-Glucamin | 0,50 |
| N-Methyl-D-Glucamin | 0,50 |
| N-Ethyl-D-Glucamin | 0,50 |
| N-Octyl-D-Glucamin | 0,50 |
| Xylit | 0,50 |
| Sorbit | 0,50 |
| Erythrit | 0,50 |
| Mannit | 0,50 |
| Glycin | 0,50 |
| Arginin | 0,50 |
| Nonansäure | 0,50 |
| Ethylcaprylat | 0,50 |
| DMSO | 0,50 |
| GESAMT | 100,00 |

2. Die Zusammensetzung nach Anspruch 1, wobei R¹ ein Wasserstoffatom oder eine C₁-C₂-Alkylgruppe ist.

3. Die Zusammensetzung nach Anspruch 1, wobei R³ eine C₇-C₉-Alkylgruppe, vorzugsweise eine C₈-Alkylgruppe, ist.

4. Die Zusammensetzung nach Anspruch 1, wobei der Gesamtanteil von Glucosamin, einem Derivat von Glucosamin oder einer Kombination davon, bezogen auf das Gesamtgewicht der Zusammensetzung, von 1,5 Gew.-% bis 2,5 Gew.-% beträgt.

5. Die Zusammensetzung nach Anspruch 1, wobei der Gesamtanteil eines oder mehrerer Tenside der Formel I, bezogen auf das Gesamtgewicht der Zusammensetzung, 0,5 Gew.-% bis 3 Gew.-% beträgt.

6. Die Zusammensetzung nach einem der Ansprüche 1 bis 5, ferner umfassend Wasser in einer Menge von 5 Gew.-% bis 98 Gew.-%, bezogen auf das Gesamtgewicht der Zusammensetzung.

7. Die Zusammensetzung nach Anspruch 6, wobei die Zusammensetzung ein Gel ist und die Zusammensetzung von 10 Gew.-% bis 95 Gew.-% Wasser, bezogen auf das Gesamtgewicht der Zusammensetzung, aufweist.

8. Die Zusammensetzung nach Anspruch 6, wobei die Zusammensetzung eine konzentrierte Spülung ist und die Zusammensetzung von 5 Gew.-% bis 65 Gew.-% Wasser aufweist.

9. Die Zusammensetzung nach Anspruch 6, wobei die Zusammensetzung eine Mundspülung ist und die Zusammensetzung eine Emulsion, die eine wässrige Phase und eine Ölphase aufweist, ist.

10. Die Zusammensetzung nach Anspruch 1, wobei die Zusammensetzung eine Zahnpasta ist und ferner ein oder mehrere Zahnschleifmittel umfasst.

11. Die Zusammensetzung nach Anspruch 1, ferner umfassend Arginin, Glycin oder eine Kombination davon in einer Menge von 0,2 Gew.-% bis 3 Gew.-%, bezogen auf das Gesamtgewicht der Zusammensetzung.

12. Die Zusammensetzung nach einem der Ansprüche 1 bis 11 zur Verwendung bei der Verhinderung, Hemmung, Beeinträchtigung oder einer beliebigen Kombination davon der Ausbildung oder Aufrechterhaltung eines Biofilms in einem oralen Gewebe, wobei die Zusammensetzung in einem Verfahren zu verabreichen ist, das umfasst:
Kontaktieren eines oralen Gewebes mit einer Zusammensetzung nach einem der Ansprüche 1 bis 11, wobei die Maßgabe von Anspruch 1 nicht gilt.

13. Die Zusammensetzung nach einem der Ansprüche 1 bis 11 zur Verwendung bei der Behandlung einer Oberfläche in der Mundhöhle eines Subjekts, wobei die Zusammensetzung in einem Verfahren zu verabreichen ist, das umfasst:
(a) Bereitstellen der Zusammensetzung nach einem der Ansprüche 1 bis 11; und
(b) Aufbringen der Zusammensetzung auf eine Oberfläche in der Mundhöhle des Subjekts, wobei die Maßgabe von Anspruch 1 nicht gilt.

14. Die Zusammensetzung zur Verwendung nach Anspruch 12 oder 13 zur Verwendung bei der Behandlung oder Vorbeugung von Zahnbelag, Zahnkaries, Zahnfleischentzündungen und/oder Parodontalerkrankungen.

15. Ein Verfahren zum Verhindern, Hemmen, Beeinträchtigen oder einer beliebigen Kombination davon der Ausbildung oder Aufrechterhaltung eines Biofilms, wobei das Verfahren *in vitro* das Kontaktieren eines Bereichs mit der Zusammensetzung nach einem der Ansprüche 1 bis 11 umfasst, wobei die Maßgabe von Anspruch 1 nicht gilt.

## Revendications

1. Composition comprenant :
de 1 % en poids à 3 % en poids au total de glucosamine, d'un dérivé de la glucosamine ou d'une combinaison de ceux-ci sur la base du poids total de la composition, dans laquelle le dérivé de la glucosamine est choisi parmi des sels de glucosamine et N-acétylglucosamine ; et
de 0,3 % en poids à 3 % en poids au total d'un ou plusieurs agents tensioactifs, sur la base du poids total de la composition, de formule I :
HOCH₂-(CHOH)ₙ-CH₂NR¹R² (I)
où
R¹ et R² sont indépendamment choisis dans le groupe constitué d'un atome d'hydrogène, d'un groupe alkyle, de C(O)R³ et de SO₂R⁴ ;
R³ et R⁴ étant indépendamment choisis dans le groupe constitué d'un groupe alkyle, d'un groupe aryle et d'un groupe aralkyle ; et
n est un nombre entier de 2 à 5,
à condition d'exclure la solution aqueuse A suivante, où DMSO est diméthylsulfoxyde :
Solution aqueuse A
| Ingrédient | % en poids |
|---|---|
| Eau | 80,10 |
| Glycérine | 10,00 |
| MÉGA-9 | 0,70 |
| Octyl glucopyranoside | 0,70 |
| N-acétyl-D-glucosamine | 0,50 |
| N-acétyl-D-galactosamine | 0,50 |
| Chlorhydrate de D-glucosamine | 0,50 |
| Sulfate de D-glucosamine | 0,50 |
| D-glucamine | 0,50 |
| N-méthyl-D-glucamine | 0,50 |
| N-éthyl-D-glucamine | 0,50 |
| N-octyl-D-glucamine | 0,50 |
| Xylitol | 0,50 |
| Sorbitol | 0,50 |
| Érythritol | 0,50 |
| Mannitol | 0,50 |
| Glycine | 0,50 |
| Arginine | 0,50 |
| Acide nonanoïque | 0,50 |
| Caprylate d'éthyle | 0,50 |
| DMSO | 0,50 |
| TOTAL | 100,00 |

2. Composition selon la revendication 1, dans laquelle R¹ est un atome d'hydrogène ou un groupe alkyle en C₁ à C₂.

3. Composition selon la revendication 1, dans laquelle R³ est un groupe alkyle en C₇ à C₉, de préférence un groupe alkyle en C₈.

4. Composition selon la revendication 1, dans laquelle le total de glucosamine, d'un dérivé de la glucosamine ou d'une combinaison de ceux-ci sur la base du poids total de la composition est comprise entre 1,5 % en poids et 2,5 % en poids.

5. Composition selon la revendication 1, dans laquelle le total d'un ou plusieurs tensioactifs de formule I, sur la base du poids total de la composition, est compris entre 0,5 % en poids et 3 % en poids.

6. Composition selon l'une quelconque des revendications 1 à 5, comprenant en outre de l'eau dans une proportion allant de 5 % en poids à 98 % en poids sur la base du poids total de la composition.

7. Composition selon la revendication 6, dans laquelle la composition est un gel et la composition contient de 10 % en poids à 95 % en poids d'eau sur la base du poids total de la composition.

8. Composition selon la revendication 6, dans laquelle la composition est un concentré de rinçage et la composition contient de 5 % en poids à 65 % en poids d'eau.

9. Composition selon la revendication 6, dans laquelle la composition est un rinçage buccal et la composition est une émulsion ayant une phase aqueuse et une phase huileuse.

10. Composition selon la revendication 1, dans laquelle la composition est un dentifrice et comprend en outre un ou plusieurs abrasifs dentaires.

11. Composition selon la revendication 1, comprenant en outre de l'arginine, de la glycine ou une combinaison de celles-ci dans une proportion de 0,2 % en poids à 3 % en poids sur la base du poids total de la composition.

12. Composition selon l'une quelconque des revendications 1 à 11 pour une utilisation dans la prévention, l'inhibition, la perturbation ou toute combinaison de celles-ci, de la formation ou du maintien d'un biofilm dans un tissu buccal, dans laquelle la composition doit être administrée dans procédé comprenant :
la mise en contact d'un tissu buccal avec une composition selon l'une quelconque des revendications 1 à 11, dans laquelle la condition de la revendication 1 ne s'applique pas.

13. Composition selon l'une quelconque des revendications 1 à 11 pour une utilisation dans le traitement d'une surface dans la cavité buccale d'un sujet, dans laquelle la composition doit être administrée dans un procédé comprenant :
(a) la fourniture de la composition selon l'une quelconque des revendications 1 à 11 ; et
(b) l'application de la composition sur une surface de la cavité buccale du sujet, dans laquelle la condition de la revendication 1 ne s'applique pas.

14. Composition pour une utilisation selon la revendication 12 ou 13 pour une utilisation dans le traitement ou la prévention de la plaque dentaire, des caries dentaires, de la gingivite et/ou des maladies parodontales.

15. Procédé de prévention, d'inhibition, de perturbation, ou toute combinaison de celles-ci, de la formation ou du maintien d'un biofilm, dans lequel le procédé comprend la mise en contact *in vitro* d'une zone avec la composition selon l'une quelconque des revendications 1 à 11, dans lequel la condition de la revendication 1 ne s'applique pas.
